Europäisches Patentamt

European Patent Office

Office Européen des brevets

(11) Veröffentlichungsnummer: **0 199 072 B1**

# (12) EUROPÄISCHE PATENTSCHRIFT

(45) Veröffentlichungstag der Patentschrift:
02.01.91 Patentblatt 91/01

(51) Int. Cl.⁵: **C07C 29/136, C07C 31/40**

(21) Anmeldenummer: **86103507.9**

(22) Anmeldetag: **15.03.86**

(54) **Verfahren zur Herstellung von 2.2.2.-Trifluoräthanol.**

Die Akte enthält technische Angaben, die nach dem Eingang der Anmeldung eingereicht wurden und die nicht in dieser Patentschrift enthalten sind.

(30) Priorität: 26.03.85 DE 3510883

(43) Veröffentlichungstag der Anmeldung:
29.10.86 Patentblatt 86/44

(45) Bekanntmachung des Hinweises auf die Patenterteilung:
02.01.91 Patentblatt 91/01

(84) Benannte Vertragsstaaten:
AT BE CH DE FR GB IT LI NL SE

(56) Entgegenhaltungen:
EP-A- 0 175 558
WO-A-82/03854
BE-A- 670 041
DE-A- 2 810 391
US-A- 3 356 747
US-A- 4 072 726

(73) Patentinhaber: Kali-Chemie Aktiengesellschaft
Postfach 220, Hans-Böckler-Allee 20
D-3000 Hannover 1 (DE)

(72) Erfinder: Willenberg, Heinrich, Dr. rer. nat.
Heinrich-Lindwedel-Strasse 16
D-3008 Garbsen 4 (DE)
Erfinder: Pohlmeyer, Wilhelm
Bunnenbergstrasse 21
D-3000 Hannover 1 (DE)
Erfinder: Rudolph, Werner, Dr. rer. nat.
Oderstrasse 38
D-3000 Hannover 71 (DE)

(74) Vertreter: Lauer, Dieter, Dr. et al
c/o Kali-Chemie AG Postfach 220
Hans-Böckler-Allee 20
D-3000 Hannover 1 (DE)

Jouve, 18, rue Saint-Denis, 75001 PARIS

## Beschreibung

Die vorliegende Erfindung betrifft ein Verfahren zur Herstellung von 2.2.2-Trifluoräthanol durch katalytische Hydrierung von Trifluoressigsäure-2.2.2.-trifluoräthylester mit elementarem Wasserstoff in der Gasphase.

2.2.2.-Trifluoräthanol besitzt eine hohe thermische Beständigkeit und - in bestimmten Mischungsverhältnissen mit anderen Stoffen - hervorstechende thermodynamische Eigenschaften. So wird es im Gemisch mit Wasser (z. B. als "Fluorinol 85" mit 85 Mol-% 2.2.2.-Trifluoräthanol und 15 Mol-% Wasser) in Wärmekraftmaschinen als Arbeitsmedium eingesetzt. Aufgrund seiner besonderen chemischen Eigenschaften dient 2.2.2.-Trifluoräthanol sowohl als Lösungsmittel (z. B. für Polyamide und Polypeptide) als auch als Reaktionskomponente (z. B. für die Herstellung von Anästhetika) ; es kann auch als Reaktionsmedium (z. B. für photolytische Reaktionen) verwendet werden.

Es ist bekannt, 2.2.2.-Trifluoräthanol durch spaltende Hydrierung von Trifluoressigsäure-2.2.2.-trifluoräthylester mit elementarem Wasserstoff in der Gasphase und in Anwesenheit katalytisch wirksamer Festkörper, also unter heterogener Katalyse, herzustellen. Dabei entstehen aus einem Mol Ester und zwei Molen Wasserstoff zwei Mole Alkohol.

So ist in DE-PS 12 71 696 ein solches, bei Temperaturen zwischen 225 und 400 °C und Drücken zwischen 1 und 5 bar (abs.) ablaufendes Verfahren beschrieben, wobei als Katalysatoren entweder Chromit-Vollkatalysatoren oder Edelmetall-Trägerkatalysatoren Verwendung finden. Als Vollkatalysatoren werden dabei z. B. Kupferchromite, Zinkchromite, Eisenchromite sowie Manganchromite bzw. Mischungen von Oxiden und Chromiten eingesetzt ; das Gewichtsverhältnis von Chromoxid zum anderen Metalloxid kann zwischen 0,5 : 1 und 10 :1 betragen.

Da nach den angeführten Beispielen die Edelmetall-Trägerkatalysatoren nur zu sehr geringen Umsätzen bzw. Selektivitäten führen, sind lediglich die Chromit-Vollkatalysatoren für eine wirtschaftliche Anwendung relevant. Mit einem aus Chromoxid und Zinkoxid bestehenden Kontakt werden allerdings völlig unbefriedigende Ergebnisse erzielt. Durch Zusatz von Kupferoxid und Calciumoxid Gew.-Verhältnisse $Cr_2O_3/ZnO = 0,4 : 1$ und $Cr_2O_3/CuO = 1,6 : 1$) wird insbesondere die Selektivität erhöht. Die relativ besten Werte hinsichtlich Laufzeit, Umsatz und Selektivität lassen sich mit einem aus Chromoxid, Kupferoxid und Bariumoxid bestehenden Katalysator erreichen (Gew.-Verhältnis $Cr_2O_3/CuO = 1,1 : 1$).

Jedoch sind die erforderlichen Reaktionstemperaturen von weit über 200°C sowie die geringen Laufzeiten von max. 27 Stunden (mit entsprechend geringen Katalysator-Ausbeuten) für eine technische Durchführung wenig brauchbar. Außerdem verbietet sich heutzutage der Einsatz solcher Chromit-Katalysatoren mit hohen Chromoxid-Gehalten aus vielerlei Gründen (z. B. Abwasserprobleme, Kanzerogenität).

Nach einer neueren Veröffentlichung (US-PS 4 072 726) läßt sich für dieselbe Umsetzung ein chromfreier Kupferkatalysator mit 50 - 100 Gew.-% Kupferoxid und 0 bis 50 Gew.-% inertem Bindemittel unter vergleichbaren Temperatur- und Druckbedingungen verwenden. Nach den angeführten Beispielen ergeben sich bei Kupferoxidgehalten zwischen 75 und 95 Gew.-% und einer Reaktionstemperatur von 235°C Laufzeiten von 53 bis 175 Stunden und Umsätze über 54% sowie eine Selektivität von mindestens 95%. Die entsprechenden Katalysator-Ausbeuten liegen zwischen 19 und 42 kg 2.2.2.-Trifluoräthanol pro kg Katalysator.

Trotz der im Vergleich zu den Ergebnissen von DE-PS 12 71 696 erheblich längeren Laufzeiten und höheren Katalysator-Ausbeuten stellen die erforderlichen, ebenfalls deutlich über 200°C liegenden Reaktionstemperaturen sowie der extrem hohe Kupferoxidgehalt, der zu einer spürbaren Verteuerung des Katalysators führt, unübersehbare Nachteile dar.

Hohe Temperaturen und hoher Kupfergehalt erhöhen außerdem die Anfälligkeit gegenüber thermischer Alterung, z. B. durch Rekristallisation und Sinterung, besonders dann, wenn das Synthesegas Spuren von Chlorverbindungen enhält (Ullmann Bd. 13, S. 545, 4. Aufl., 1976). Dies ist aber in technischen Verfahren stets der Fall, wenn der zu hydrierende Trifluoressigsäure-2.2.2.-trifluoräthylester, wie eingangs von US-PS 4 072 726 beschrieben, durch Umsetzung des entsprechenden Säurechlorids mit 2.2.2.-Trifluoräthanol hergestellt wird.

Aufgabe der vorliegenden Erfindung ist es, ein Verfahren zur Herstellung von 2.2.2.-Trifluoräthanol durch katalytische Hydrierung von Trifluoressigsäure-2.2.2.-trifluoräthylester mit elementarem Wasserstoff in der Gasphase zur Verfügung zu stellen, das die geschilderten Nachteile des Standes der Technik überwindet.

Die Lösung dieser Aufgaben erfolgt durch Bereitstellung der durch die Patentansprüche gekennzeichneten Verfahren.

Das erfindungsgemäße Verfahren geht gattungsgemäß aus von einem Verfahren zur Herstellung von 2.2.2.-Trifluoräthanol durch katalytische Hydrierung von Trifluoressigsäure-2.2.2.-trifluoräthylester mit elementarem Wasserstoff in der Gasphase in Gegenwart eines Zink- und Kupferoxid enthaltenden Katalysators und ist dadurch gekennzeichnet, daß der Katalysator aus den Oxiden von Zink- und Kupfer besteht, sowie gegebenenfalls Oxide von Eisen, Chrom, Mangan, Calcium, Aluminium und/oder Silicium enthält, wobei die Summe aller Oxide stets 100 Gew.-% ist und das Gewichtsverhältnis von Chromoxid zur Summe der übrigen Bestandteile 0 bis 0,1 : 1, vorzugsweise 0 bis 0.05 : 1 ist und der Katalysator 10 bis 90 Gew.% Zinkoxid und 10 bis 90 Gew.-% Kupfer oxid enthält.

Bevorzugt enthält der Katalysator 10 bis 60 Gew.-% Zinkoxid und 10 bis 60 Gew.-% Kupferoxid.

In einer Verfahrensvariante kann der Katalysator allein aus Kupferoxid und Zinkoxid bestehen.

In einer anderen Variante enhält der Katalysator zusätzlich noch Oxide von Eisen, Chrom, Mangan, Calcium, Aluminium und/oder Silicium, vorzugsweise Oxide von Chrom, Calcium, Aluminium und/oder Silicium. In dieser Variante ist vorgesehen, daß der Aluminiumoxid-Anteil insbesondere im Bereich von 0 bis 40 Gew.-% liegt, während der Anteil der übrigen Oxide bei jeweils 0 bis 5 Gew.-% liegt.

Besonders bevorzugt sind Kontakte, bei denen Kupfer- und Zinkoxid gemeinsam über 50 Gew.-% des Katalysators ausmachen.

Für das erfindungsgemäße Verfahren können an sich bekannte Katalysatoren verwendet werden. Bevorzugt werden jene Katalysatoren, die ausgehend von einem Mischkristall-Vorläufer mit gemeinsamen, leicht zersetzlichen Anionen, wie z. B. Carbonat, Formiat oder Oxalat, hergestellt werden. Dadurch läßt sich ein hoher Dipersionsgrad der aktiven Komponenten erreichen (Ullmann, Bd. 13, S. 528 u. 559, 4. Aufl. 1976).

Kupfer-Zink-Katalysatoren werden bekanntlich für die Herstellung von Methanol aus Kohlenmonoxid, Kohlendioxid und Wasserstoff im sogenannten Niederdruckverfahren eingesetzt. Die dabei angewandten Temperaturen bzw. Drücke liegen im Bereich von 230 bis 280°C bzw. 50 bis 100 bar (Ullmann, Bd. 16, S. 624 ff., 4. Aufl., 1976). Es sind nirgendwo Hinweise darauf zu finden, daß derartige Katalysatoren für die spaltende Hydrierung von Estern geeignet sind. Ebenso war nicht zu vermuten, daß der Arbeitsbereich dieser Kontakte unterhalb von 230°C bzw. 50 bar liegen könnte.

Überraschenderweise eignen sich Kupfer-Zink-Katalysatoren der beschriebenen Art in hervorragender Weise für die katalytische Gasphasen-Hydrierung von Trifluoressigsäure-2.2.2.-trifluoräthylester mit elementarem Wasserstoff, und zwar gerade im Temperaturbereich zwischen 100 und 230°C und im Druckbereich zwischen 1 und 5 bar (abs.).

In einer besonderen Ausführungsform werden vorteilhafterweise Temperaturen zwischen 120 und 210°C sowie Drücke zwischen 1,5 bis 2,5 bar (abs.) angewendet. Dabei wird zum Zweck einer Umsatzoptimierung während der Laufzeit der Katalysatorschüttung die Reaktortemperatur kontinuierlich gesteigert, während der Druck im wesentlichen konstant gehalten wird.

Die Leerrohrverweilzeit soll unter den gewählten Temperatur- und Druckverhältnissen 5 bis 50 Sekunden, (bevorzugt 10 bis 25 Sekunden) betragen.

Das Molverhältnis von Wasserstoff zu Ester kann unterstöchiometrisch, stöchiometrisch oder überstöchiometrisch sein, und zwar soll es die Relation 1,5 : 1 bzw. 3,5 : 1 nicht unter- bzw. überschreiten. In der Anfahrphase wird man wegen der besonders hohen Aktivität des frischen, noch unverbrauchten Kontaktes mehr den unterstöchiometrischen Bereich wählen und die Relation erst im Laufe der Zeit steigern. Das während der Hauptproduktionsphase bevorzugte Molverhältnis liegt vorteilhafterweise zwischen 2 : 1 und 3 : 1.

Das erfindungsgemäße Verfahren umfaßt auch eine zyklisch arbeitende Verfahrensvariante, bei der Hydrierung und Katalysatorregenerierung abwechselnd durchgeführt werden. Diese Variante ist dadurch gekennzeichnet, daß man das Verfahren zyklisch durchführt indem man

a) die Hydrierung gegebenenfalls unter fortschreitender Temperatursteigerung bis zu nachlassendem Umsatz führt, dann

b) den Katalysator in situ mit Wasser regeneriert und anschließend

c) einen neuen Hydrier/Regenerations-Zyklus gemäß den Stufen a) und b) beginnt.

Zur Regenerierung des Katalysators wird Wasser in flüssiger Form bzw. Dampfform, vorzugsweise in Dampfform eingesetzt. Der Einsatz von Wasser in flüssiger Form zur Katalysatorreaktivierung bezieht sich nach dem Stand der Technik auf eine durch Vergiftung (poisoning) verursachte Desaktivierung, die im allgemeinen zumindest partiell reversibel ist.

Jedoch resultiert auch nach einer vollständigen Entfernung des Chlorids (von geringsten Chloridanteilen im eingesetzten Trifluoressigsäure-2.2.2.-trifluoräthylester herrührend) mittels polarer organischer Lösungsmittel wie 2.2.2.-Trifluoräthanol oder Methanol bei den hier beschriebenen Kupfer-Zink-Katalysatoren keine Wiederbelebung des Kontaktes. Überraschenderweise bewirkt erst die zusätzliche (simultan bzw. sukzessiv ausgeführte) oder alleinige Behandlung mit Wasser oder Wasserdampf eine Regenerierung des Katalysators. Diese beruht vermutlich darauf, daß durch Alterung (aging) bedingte Wasserabspaltun-

3

gen zumindest teilweise rückgängig gemacht werden.

Vorteilhafterweise wird die Regenerierung in situ, d. h. im Reaktionsrohr, durchgeführt, da dann die Entleerung des Reaktors und die ggf. erforderliche Oberflächen-Passivierung des pyrophoren reduzierten Kontaktes durch Oxidation entfallen kann. Es ist aber selbstverständlich auch möglich, die Regenerierung nach Überführung des gealterten Kontaktes in ein eigenes Behältnis, z. B. einen sogenannten Regenerator, vorzunehmen.

Die folgenden Beispiele sollen das Verfahren näher beschreiben ohne seinen Schutzumfang zu beschränken :

Beispiele

In den Beispielen wurden im Handel erhältliche Katalysatoren auf Carbonatbasis (Tabletten von ca. 5 mm Höhe und Durchmesser) der in der Tabelle angegebenen Zusammensetzung verwendet. Bei der Angabe der Zusammensetzung des Katalysators werden die Anteile an Metallen als Oxide angegeben. Nur in Spuren vorliegende Anteile (z. B. Natrium, Kalium) werden dabei ebensowenig berücksichtigt, wie Anionen-Anteile (z. B. Carbonat, OH) oder Wasser. Nach Befüllung des mit einem Heizmantel versehenen Reaktionsrohres wurde der Kontakt im Rohr nach Vorschrift aktiviert, indem er bei Temperaturen bis zu etwa 250°C vorsichtig mit Wasserstoff-Stickstoff-Gemischen (1 bis 100 Vol.-% $H_2$) reduziert wurde.

Reaktion :

Danach erfolgte unter gemeinsamer Dosierung von verdampftem Trifluoressigsäure-2.2.2.-trifluoräthylester (in Form seines Azeotropes mit 2.2.2.-Trifluoräthanol) und elementarem Wasserstoff die Umsetzung der beiden Reaktanden zu 2.2.2.-Trifluoräthanol unter den in der nachfolgenden Tabelle genannten Reaktionsbedingungen. Während der Laufzeit wurde die Reaktionstemperatur durch Erhöhung der Temperatur des das Reaktionsrohr über den Heizmantel erwärmenden bzw. abkühlenden Ölbades kontinuierlich gesteigert.

Dabei durchlief der Umsatz ein Maximum, während die Selektivität zwischen 95 und 100 Mol-% schwankte. Im Verlauf der Umsetzung wurde das Molverhältnis $H_2$/Ester entsprechend der Aktivitätsabnahme des Katalysators erhöht. In der Tabelle ist das durchschnittliche Molverhältnis angegeben.

Regeneration :

In den Beispielen 2.1 bzw. 3.1 und 3.2 wurde nach der Reaktion jeweils eine Regeneration durchgeführt, um einen weiteren (zweiten oder dritten) Zyklus mit derselben Schüttung anschließen zu können. Dieser Prozeß erfolgte stets in situ, d. h. im Reaktionsrohr, wobei die flüssigen Medien bei Raumtemperatur unter Kreislaufführung durch die Schüttung gepumpt wurden, und das gasförmige Medium ($H_2$ O Dampf) unter Beheizung des Reaktionsrohres durch die Schüttung geführt wurde. In den flüssigen Medien wurde die aufgrund Massenbilanz erwartete Chloridmenge wiedergefunden.

Im Anschluß an Beispiel 2.1 wurde mit einem flüssigen Gemisch aus Trifluoräthanol/Wasser bei Raumtemperatur regeneriert, im Anschluß an Beispiel 3.1 mit Wasser bei Raumtemperatur und im Anschluß an Beispiel 3.2 mit Wasserdampf im Bereich der Reaktionstemperatur. Ähnliche Regenerationsversuche allein mit Trifluoräthanol oder Methanol führten zu keiner befriedigenden Regenerierung des Katalysators.

## Tabelle 1:

| Beispiele<br><br>Kenndaten | 1 | 2.1. | 2.2. | 3.1. | 3.2. | 3.3. |
|---|---|---|---|---|---|---|
| **Katalysator:** | | | | | | |
| Gew.-% CuO | 44 | 43 | wie 2.1. | wie 2.1. | wie 2.1. | wie 2.1. |
| Gew.-% ZnO | 21 | 54 | " | " | " | " |
| Gew.-% $Al_2O_3$ | 31 | 3 | " | " | " | " |
| Gew.-% $Cr_2O_3$ | 4 | -- | " | " | " | " |
| Schüttungsnr. | 1 | 2 | 2 | 3 | 3 | 3 |
| Zyklusnr. | 1 | 1 | 2 | 1 | 2 | 3 |
| **Reaktion:** | | | | | | |
| Gew.-ppm Chlorid*) | 200 | 200 | 200 | 20 | 20 | 20 |
| Druck (bar abs.) | 2 | 2 | 2 | 2 | 2 | 2 |
| Verweilzeit (s)**) | 11 – 15 | 12 – 15 | 12 – 15 | 12 – 15 | 12 – 15 | 12 – 13 |
| Molverh. $H_2$/Ester | 2,9 | 2,8 | 2,8 | 2,8 | 2,8 | 2,8 |
| Temperatur (°C) | 125 – 200 | 115 – 210 | 150 – 200 | 120 – 200 | 150 – 204 | 155 – 209 |
| max. Umsatz (Mol-%) | 55 | 72 | 45 | 77 | 45 | 39 |
| Laufzeit (h) | 121 | 186 | 121 | 221 | 116 | 64 |
| Katalysator-Ausbeute in (kg TFE/kg Kat.) | 44 | 44 | 25 | 67 | 20 | 13 |

*) im eingesetzten Ester bzw. Azeotrop      **) Leerrohrverweilzeit unter gegeb. Druck u. Temp.

KCDD97TAB

EP 0 199 072 B1

## Ansprüche

1. Verfahren zur Herstellung von 2.2.2 -Trifluoräthanol durch katalytische Hydrierung von Trifluoressigsäure-2.2.2.- Trifluorethylester mit elementarem Wasserstoff in der Gasphase in Gegenwart eines Zink- und Kupferoxid enthaltenden Katalysators, dadurch gekennzeichnet, daß der Katalysator aus den Oxiden von Zink und Kupfer besteht sowie gegebenenfalls Oxide von Eisen, Chrom, Mangan, Calcium, Aluminium und-/oder Silicium enthält, wobei die Summe aller Oxide stets 100 Gew.-% ist, das Gewichtsverhältnis von Chromoxid zur Summe der übrigen Bestandteile 0 bis 0,1 : 1 ist und der Katalysator 10 bis 90 Gew.-% Zinkoxid und 10 bis 90 Gew.-% Kupferoxid enthält.

2) Verfahren nach Anspruch 1, dadurch gekennzeichnet, daß das Verhältnis von Chromoxid zur Summe der übrigen Bestandteile 0 bis 0,05 : 1 ist.

3) Verfahren nach einem der vorhergehenden Ansprüche, dadurch gekennzeichnet, daß der Katalysator 10 bis 60 Gew.-% Zinkoxid und 10 bis 60 Gew.-% Kupferoxid enthält.

4) Verfahren nach einem der vorhergehenden Ansprüche, dadurch gekennzeichnet, daß der Katalysator 0 bis 40 Gew.-% Aluminiumoxid enthält.

5) Verfahren nach einem der vorhergehenden Ansprüche, dadurch gekennzeichnet, daß der Katalysator 0 bis 5 Gew.-% Eisen-, Chrom-, Mangan-, Calcium- und/oder Silicium-Oxid, vorzugsweise Chrom-, Calcium und/oder Silicium-Oxid enthält.

6) Verfahren nach einem der vorhergehenden Ansprüche, dadurch gekennzeichnet, daß die Reaktionstemperatur zwischen 100 und 230°C, vorzugsweise 120 und 210°C liegt.

7) Verfahren nach einem der vorhergehenden Ansprüche, dadurch gekennzeichnet, daß der Gesamtdruck 1 bis 5 bar (absolut) vorzugsweise 1,5 bis 2,5 bar (absolut) beträgt.

8) Verfahren nach einem der vorhergehenden Ansprüche, dadurch gekennzeichnet, daß die Leerrohrverweilzeit unter Reaktionsbedingungen 5 bis 50 Sekunden, vorzugsweise 10 bis 25 Sekunden beträgt.

9) Verfahren nach einem der vorhergehenden Ansprüche, dadurch gekennzeichnet, daß das Molverhältnis von Wasserstoff zu Ester 1,5 : 1 bis 3,5 : 1, vorzugsweise 2 : 1 bis 3 : 1 beträgt.

10) Verfahren nach einem der vorhergehenden Ansprüche, dadurch gekennzeichnet, daß man das Verfahren zyklisch durchführt, indem man

a) die Hydrierung gegebenenfalls unter fortschreitender Temperatursteigerung bis zu nachlassendem Umsatz führt, dann

b) den Katalysator in situ mit Wasser regeneriert und anschließend

c) einen neuen Hydrier/Regenerations-Zyklus gemäß den Stufen a) und b) beginnt.

## Claims

1. Process for the preparation of 2,2,2-trifluoroethanol by catalytic hydrogenation of trifluoroacetic acid-2,2,2-trifluoroethyl ester with elemental hydrogen in the gas phase in the presence of a catalyst containing zinc oxide and copper oxide, characterised in that the catalyst consists of the oxides of zinc and copper, and also optionally oxides of iron, chromium, manganese, calcium, aluminium and/or silicon, with the total of all the oxides always being 100% by weight, the weight ratio of chromium oxide to the total of the other constituents being 0 to 0.1 : 1 and the catalyst containing 10 to 90% by weight zinc oxide and 10 to 90% by weight copper oxide.

2. Process according to Claim 1, characterised in that the ratio of chromium oxide to the total of the other constituents is 0 to 0.05 : 1.

3. Process according to one of the preceding Claims, characterised in that the catalyst contains 10 to 60% by weight zinc oxide and 10 to 60% by weight copper oxide.

4. Process according to one of the preceding Claims, characterised in that the catalyst contains 0 to 40% by weight aluminium oxide.

5. Process according to one of the preceding Claims, characterised in that the catalyst contains 0 to 5% by weight iron oxide, chromium oxide, manganese oxide, calcium oxide and/or silicon oxide, preferably chromium oxide, calcium oxide and/or silicon oxide.

6. Process according to one of the preceding Claims, characterised in that the reaction temperature is between 100 and 230°C, preferably 120 and 210°C.

7. Process according to one of the preceding Claims, characterised in that the total pressure is 1 to 5 bar (absolute), preferably 1.5 to 2.5 bar (absolute).

8. Process according to one of the preceding Claims, characterised in that the empty pipe dwell time under reaction conditions is 5 to 50 seconds, preferably 10 to 25 seconds.

9. Process according to one of the preceding Claims, characterised in that the molar ratio of hydrogen to ester is 1.5 : 1 to 3.5 : 1, preferably 2 : 1 to 3 : 1.

10. Process according to one of the preceding Claims, characterised in that the process is carried out cyclically, by

a) carrying out the hydrogenation optionally with progressive temperature increase until conversion decreases, then

b) regenerating the catalyst in situ with water and then

c) beginning a new hydrogenation/regeneration cycle according to stages a) and b).

## Revendications

1. Procédé de préparation de 2,2,2-trifluoroéthanol par hydrogénation catalytique de 2,2,2-trifluoroéthylester de l'acide trifluoroacétique avec de l'hydrogène élémentaire en phase gazeuse en présence d'un catalyseur contenant de l'oxyde de zinc et de l'oxyde cuivre, caractérisé en ce que le catalyseur se compose des oxydes de zinc et de cuivre, et contient éventuellement des oxydes de fer, de chrome, de manganèse, de calcium, d'aluminium et/ou de silicium, la somme de tous les oxydes étant toujours de 100% en poids, le rapport pondéral de l'oxyde de chrome à la somme des autres composants étant de 0 à 0,1 :1, et en ce que le catalyseur contient de 10 à 90% en poids d'oxyde de zinc et de 10 à 90% en poids d'oxyde de cuivre.

2. Procédé selon la revendication 1, caractérisé en ce que le rapport de l'oxyde de chrome à la somme des autres composants est de 0 à 0,05 :1.

3. Procédé selon l'une des revendications précédentes, caractérisé en ce que le catalyseur contient de 10 à 60% en poids d'oxyde de zinc et de 10 à 60% en poids d'oxyde de cuivre.

4. Procédé selon l'une des revendications précédentes, caractérisé en ce que le catalyseur contient de 0 à 40% en poids d'oxyde d'aluminium.

5. Procédé selon l'une des revendications précédentes, caractérisé en ce que le catalyseur contient de 0 à 5% en poids d'oxyde de fer, de chrome, de manganèse, de calcium et/ou de silicium, de préférence, d'oxyde de chrome, de calcium et/ou de silicium.

6. Procédé selon l'une des revendications précédentes, caractérisé en ce que la température réactionnelle se situe entre 100 et 230°C, de préférence 120 et 210°C.

7. Procédé selon l'une des revendications précédentes, caractérisé en ce que la pression totale s'élève à 1 à 5 bars (absolus), de préférence 1,5 à 2,5 bars (absolus).

8. Procédé selon l'une des revendications précédentes, caractérisé en ce que le temps de séjour dans le tube creux dans les conditions de la réaction est de à 5 à 50 secondes, de préférence de 10 à 25 secondes.

9. Procédé selon l'une des revendications précédentes, caractérisé en ce que le rapport molaire de l'hydrogène à l'ester est de à 1,5 :1 à 3,5 :1, de préférence de 2 :1 à 3 :1.

10. Procédé selon l'une des revendications précédentes, caractérisé en ce qu'on conduit le procédé de façon cyclique

a) en conduisant l'hydrogénation éventuellement en poursuivant l'accroissement de la température jusqu'à ce que le taux de transformation baisse, puis

b) en régénérant le catalyseur in situ avec de l'eau et ensuite

c) en commençant un nouveau cycle d'hydrogénation/régénération selon les étapes a) et b).